# EUROPEAN PATENT APPLICATION

(11) **EP 4 060 342 A1**
(43) Date of publication of application: **21.09.2022**
(21) Application number: 21163459.7
(22) Date of filing: 18.03.2021
(51) Int. Cl.: G01N 33/558, G01N 33/561, G01N 33/569

(54) **LATERAL FLOW ASSAYS FOR DIAGNOSIS OF INFECTIOUS DISEASES**

(71) Applicant: AegirBio AB, 223 70 Lund (SE)
(72) Inventor: TAKWA, Mohamad, 22 472 Lund (SE); FRAGKOU, Vasiliki, Kettering, NN15 5GQ (GB); WRIGHT, Milo, Thomas, Felmersham, MK43 7HN (GB)
(74) Representative: Høiberg P/S

(57) **Abstract**

The present disclosure relates to lateral flow devices and methods for detecting a target compound in a sample and for diagnosis of infectious diseases.

## Description

### Technical field

The present invention relates to lateral flow devices and methods for detecting a target compound in a sample and for diagnosis of infectious diseases.

### Background

A lateral flow device is a simple-to-use diagnostic device for confirming the presence or absence of a specific target analyte, typically a pathogen or a biomarker in humans or animals, or contaminants in water supplies, foodstuffs, or animal feeds. A lateral flow device requires minimal training to operate and can be qualitative and read visually, or provide data when combined with reader technology.

Lateral flow devices are widely used in human health for point-of-care and point-of-need testing. They can be performed by a healthcare professional or by the patient in a wide range of settings, such as a laboratory, clinic or home. The versatile nature of lateral flow devices have rendered them being used in a number of other industry sectors including pharma, environmental testing, animal health, food and feed testing, and plant and crop health.

A typical lateral flow device is composed of several porous materials, onto which assay reagents are striped, sprayed, or spotted, then dried for storage in spatially distinct locations, and through which analyte, in a clinical or environmental sample, and assay reagents are transported by capillary action. Commonly, a sample pad is used for receiving a sample, localized at one end of the strip. The sample will then flow by capillary action through a conjugate pad, where conjugates, comprising indicator particles (such as colloidal gold) together with detection/capture molecules specific for the target compound, have been deposited and dried. When the sample gets in contact with the dried conjugates, they dissolve and bind to the target compound in the sample, thereby forming a complex. This complex continues to flow through the membrane of the lateral flow device, where there are one or more test lines for the target compounds, The test lines contain target-specific molecules, e.g. antibodies. For a sandwich assay, a visually present test line indicates that the target compound has bound to the test line, and that it therefore is present in the sample. For a competitive assay, it is instead the absence of a visually present test line that indicates the presence of the target compound in the sample. Additionally, the lateral flow device typically comprises a control line, which binds and immobilizes conjugates unbound to the target compound. A visually present control line indicates that the test has operated correctly.

The simplicity and versatility of lateral flow devices has allowed them to gain widespread use in the diagnosis of a wide range of diseases and conditions. However, one challenge with lateral flow devices is a limited sensitivity compared to other analytical methods, for example other antibody-based tests such as ELISA. These t are typically associated with a superior sensitivity compared to conventional lateral flow devices.

One important factor for the limited sensitivity, as compared to the other types of analytical methods, is that the format of the lateral flow devices poses a significant limitation on the sample. While lateral flow devices typically are limited to analysis of urine, blood, or water samples, other kinds of samples may in many cases be more readily accessible or may comprise a higher amount of the target analyte. However, the incompatibility between conventional lateral flow devices and specific sample types results in that these samples cannot be measured using conventional lateral flow devices, or at best, that the sensitivity and specificity is significantly lower than comparable analytical methods.

Hence, there is a strong need for lateral flow devices and related methods, that are compatible with a multitude of sample types, for increased sensitivity and specificity of the results, and convenience to the patient.

### Summary

The present inventors have realized methods and lateral flow devices for analysis of samples that allows for an increased sensitivity, specificity and convenience to the patient. The methods and devices use samples that are more readily accessible and that, together with specific lateral flow devices and/or sample processing, allow for a rapid and sensitive measurement of a target analyte.

Therefore, in a first aspect, the invention relates to method for detecting the presence of at least one target compound in a sample, the method comprising:
- providing a buffer solution;
- providing a lateral flow device comprising
   i. a membrane layer, comprising at least one test line, the at least one test line including an immobilized capturing agent for capturing the at least one target compound in the sample;
   ii. a sample loading zone for receiving the sample, the sample loading zone in fluidic connection with the test line;
- mixing the sample with the buffer solution, wherein the volumetric ratio between the buffer solution and the sample is between 5 parts buffer solution and 1 part sample and 15 part buffer solution and 1 part sample, thereby obtaining a sample solution;
- applying the sample solution to the sample loading zone;
- measuring the presence of the at least one target compound in the sample.

In a preferred embodiment of the present disclosure, the sample is a saliva sample. Obtaining saliva samples is associated with limited inconvenience for the patient, as compared to collection of other sample types, such as a nasopharyngeal sample. For example 0.1-1 ml of saliva may be rapidly collected from the mouth of the patient and has been shown to allow for sensitive quantitative and/or qualitative measurements of a target analyte in a sample.

The sample, preferably a saliva sample, is mixed with a buffer solution. The buffer solution may be selected to decrease non-specific binding when performing the test, dissolve components, such as mucous, of the sample, stabilizing the sample, and/or to adjust the viscosity of the sample.

The method of the present disclosure, as well as the disclosed kits provide several advantages over previously known methods. One advantage is that sensitive and highly specific measurements are possible without a need for a running buffer. A running buffer may be used, but the method and kits of the present disclosure can be used successfully without the need of adding a running buffer. Another advantage of the method and kits of the present disclosure is that aggregation in the sample is avoided by pre-treating the sample with a suitable buffer solution to obtain a sample solution. A further advantage of the method and kits of the present disclosure is that non-specific binding (NSB) to the immobilized capturing agent is minimized or even completely avoided. Furthermore, the method and kits of the present disclosure allow a more efficient detection of the presence of a target compound in a sample by minimizing, such as reducing, the waiting time required for the sample solution to migrate to the membrane layer comprising the immobilized capturing agent.

The lateral flow device may comprise conjugates, typically provided in a dry state as part of the conjugate zone. Additionally, or alternatively, the conjugates may be provided as part of a buffer solution and/or a running buffer. In such a case, the running buffer or buffer solution may also be referred to as a conjugate solution. Typically the conjugates comprise a label and at least one conjugate protein binders. The conjugates may thereby be configured to bind to the one or more target compounds and, thereby provide an indication of the presence of the target compound in the sample.

Depending on the configuration of the lateral flow device, the indicator for the presence of the target compound may be a visually observable line at the test line, or the absence of a visually observable line at the test line, preferably in combination with a visually observable line at the control line. Typically a sandwich assay generates a visually observable line, if the target compound is present in the sample, while a competitive assay would result in no visually observable line at the test line. However, the lateral flow device may be configured with a combination of a number of sandwich assay test lines and a number of competitive test lines. In general, it may be a preference that the control line is visually present for indicating that the test has operated correctly

The conjugate zone is typically located near an end of the lateral flow device, such as a proximate end. By this arrangement, the proximate end may be put in contact with a buffer solution, potentially comprising the conjugates. The solution will thereafter, by capillary action, flow through the device, and may act to drive the sample towards the test line, while maintaining a control of the interactions between the sample and the conjugates. Preferably, the sample reaches the test line before the conjugates, thereby the capturing agent may bind to the target compound, if provided in a sandwich assay format, and the conjugates may thereafter bind and form a visually observable indication of the presence of the target compound.

In a further aspect, the invention relates to a kit for detecting the presence of a target compound in a sample, the kit comprising:
- a buffer solution for diluting the sample, thereby forming a sample solution, wherein said buffer solution comprises sodium chloride, bovine serum albumin, and a borate buffer; and
- a lateral flow device comprising:
   i. a membrane layer, comprising at least one test line, the at least one test line comprising an immobilized capturing agent for capturing the at least one target compound in the sample; and
   ii. a sample loading zone for receiving the sample solution, the sample loading zone in fluidic connection with the test line.

Depending on the configuration of the lateral flow device, the kit may advantageously be provided, in the dry unused state, with a sample loading zone comprising conjugates. The conjugates may for example have been striped, sprayed, or spotted, then dried on the conjugate zone before use of the kit. When in use, a fluid, such as the sample solution, preferably interacts with the conjugates and may further act to displace the conjugates towards the one or more test lines, a control line, and/or an absorption pad, if present.

Additionally or alternatively, the conjugates may be provided as part of the sample solution or a running buffer. The kit may for example comprise a receptacle retaining the buffer solution, and may further be configured such that the buffer solution is applied to the lateral flow device by exposing the proximate end of the lateral flow device to the buffer solution.

The kit may further comprise a running buffer, i.e. a chase solution, preferably provided together with a receptacle containing said running buffer. The running buffer is advantageously added to the device following addition of the sample solution. The fluid may act to transport the sample to the test line without the formation of a significant amount of complexes comprising the conjugate and the target compound at undesirable locations.

### Description of Drawings

**Fig. 1** shows a schematic illustration of a lateral flow device according to a specific embodiment of the present disclosure.
**Fig. 2** shows a schematic illustration of a lateral flow device according to a specific embodiment of the present disclosure.
**Fig. 3** shows schematic illustration of a lateral flow device and the use thereof to detect the presence of a target analyte in a sample, according to a specific embodiment of the present disclosure.
**Fig. 4** shows schematic illustrations of possible outcomes of measurements of a sample by a lateral flow device, according to a specific embodiment of the present disclosure.

### Detailed description

One embodiment the present invention relates to a lateral flow device for detecting the presence of a target compound in a sample. The device may comprise a membrane layer and a loading zone. Said loading zone may be provided as a part of the membrane layer or may be a separate component, but preferably in fluidic contact with the membrane layer. The membrane layer may comprise one or more test lines comprising immobilized capturing agent(s) specific for one or more target compounds. Typically, each test line comprises a specific type of capturing agent. Thereby, each test line is directed towards a specific target compound. Furthermore, the lateral flow device may comprise a loading zone. Said loading zone may comprise a conjugate zone and a sample loading zone. Said loading zone may comprise a sample loading zone and no conjugate zone, as the methods disclosed herein can successfully detect the presence of a target compound also without using conjugates. The sample loading zone and the conjugate zone may be provided as a single unit, comprising a reagent zone and/or a reagent pad. However, the sample loading zone and the conjugate zone may further be provided as separate units, preferably still in fluidic contact. It is a preference that the sample loading zone is located, along the axial length of the lateral flow device, between the conjugate zone and the at least one test line. Typically the lateral flow device comprises an absorption pad at a distal end of the lateral flow device.

If a conjugate zone is present, in an embodiment of the present disclosure, the sample loading zone is located between the conjugate zone and the test line, thereby allowing the sample to flow before the conjugate, which will decrease the risk of the sample and conjugate mixing and potentially aggregating, thereby increasing the risk of them not reaching the test lines.

As used herein "conjugate" or "conjugated" refers to two molecules that are bound to each other. For example, it may refer to a particle, such as gold, latex or any other colloidal material, bound to the target compound or a protein containing the epitope or epitopes of the target compound. The particle can also be bound to other detection/capture molecules specific for the target compound.

As used herein, the singular forms "a", "an" and "the" include plural referents unless the context clearly states otherwise. Thus, for example, reference to "an antibody" includes a plurality of such antibodies.

The term "aptamer" as used herein refers to a single-stranded oligonucleotide (single-stranded DNA or RNA molecule) that can bind specifically to its target with high affinity. The aptamer can be used as a biosensor element capable of binding to a molecule in a detection/analysis system, and thus has been recognized as a substitutive for antibody. Particularly, the aptamers can be used as molecules targeting various organic and inorganic materials, including toxins, unlike antibodies, and once an aptamer binding specifically to a certain material is isolated, it can be consistently reproduced at low costs using automated oligomer synthesis methods. Further, the term "affimer" refers to small, engineered non-antibody binding proteins that can bind target molecules and are designed to mimic the molecular recognition characteristics of monoclonal antibodies. Affimer reagents are suitable for use in for example biosensors and point-of-care diagnostics.

As used herein "polymer" refers to macromolecular materials having at least five repeating monomeric units, which may or may not be the same. The term polymer, as used herein, encompasses homopolymers and copolymers. A molecularly imprinted polymer is a polymer that has been processed using the molecular imprinting technique which leaves cavities in the polymer matrix with an affinity for a chosen template molecule. Molecularly imprinted polymers possess the most important features of biological receptors - recognition. Molecularly imprinted polymers can comprise cross linked polymers. They can also comprise amorphous metal oxides or zeolites. Metal oxides and zeolites can be imprinted using a variety of known techniques. In some cases, the cavities or pores produced are an induced fit for polymers of the imprinting molecules.

In one embodiment of the present disclosure, the conjugate zone of said lateral flow device comprises conjugates. For example, the conjugate zone, in the dry unused state, may comprise at least one type of conjugate. The conjugates may thereby have been preloaded on the device, such as on a conjugate pad. Conjugates may for example be applied to the conjugate zone with the use of an air jet dispensing platform or by immersion. Several machines with hollow fibre dispensers used to stripe nitrocellulose membranes can also be configured with an air jet spray apparatus to dispense the conjugates onto the conjugate zone.

In a further embodiment of the present disclosure, the conjugate zone does not comprise conjugates.

In an embodiment of the present disclosure a conjugate comprises a label and at least one conjugate protein binder, such as multiple conjugate protein binders. The conjugate protein binders may be configured for binding to an immobilized capturing agent, at any of the test lines. In such cases, the presence of a target compound at a test line, may result in the conjugate not being able to bind to the immobilized capturing agent. The conjugate protein binder may further be configured to bind to a target compound. In such cases the absence of a target compound at a test line may result in the conjugate not being immobilized at the test line. The conjugate protein binder, in combination with the capturing agent and the target compound, may be configured for binding according to a sandwich assay or a competitive assay. It should be appreciated that a lateral flow device comprising multiple lines, such as test lines and control lines, may comprise lines configured for sandwich assay format and lines configured for competitive assay format.

In a further embodiment of the present disclosure, the label is a metal nanoparticle, such as a gold nanoparticle, and/or a coloured latex particle. A person skilled in the art will appreciate that the capturing agent may further be any other type of sol-based material. A sol is a colloid made out of very small solid particles in a continuous liquid medium. Colloidal gold is the most widely used label in lateral flow devices, it has an intense colour and no development process is needed for visualization. Moreover, it has high stability in both liquid and dried forms. Another popular label is latex, which can be tagged with a variety of detector reagents such as coloured or fluorescent dyes, and magnetic or paramagnetic components. As latex can be produced in multiple colours, it has an application in multiplex assays, which require discrimination between numerous lines. Carbon, such as carbon nanotube, fluorescent labels, or enzymatic modification of the labels, may also be used to improve the sensitivity of the assay. The label may further be a fluorescent nanoparticle, such as a quantum dot.

In a further embodiment of the present disclosure, the conjugate protein binder is selected from the list including an antibody, an Ig unit, an aptamer, an affimer, an RNA molecule, a DNA molecule, an organic polymer, or a fragment thereof. The conjugate may comprise a single or multiple conjugate protein binders. In yet a further embodiment of the present disclosure, the organic polymer is a molecularly imprinted polymer.

In another embodiment of the present disclosure, the membrane layer of said lateral flow device comprises multiple test lines for capturing and/or detecting the presence of multiple target compounds in the sample. A multiplexed lateral flow device preferably comprises multiple test lines, wherein each test line comprises a binding agent for a specific target compound. The lateral flow device may thereby for example detect the presence of pathogens, such as virus or bacteria, while simultaneously having test lines comprising binding agents directed at capturing and/or detecting the presence of IgG and IgM in the sample. The presence of IgG and IgM should here be understood as an elevated concentration of IgG and IgM, as to normal reference values, such as reference values for the patient group of the individual which is diagnosed.

This is important as when a patient undergoes an infection the pathogen, such as a virus, goes through a stage of replication and will elicit an immune response. In a first stage, the immune system produces IgM antibodies. Once the infection is under control, the immune system commences producing a more effective and long-term IgG response in order to eradicate the infection and prevent a future re-infection.

Thereby, in general a positive IgM test indicates that the subject has been infected and that the subject's immune system has started responding to the pathogen. When IgM is detected the subject may still be infected, or the subject may have recently recovered from the pathogen infection.

In most subjects IgG is developed within seven to ten days after manifestation of symptoms of the pathogen infection has begun, and the IgG antibodies remain in the blood after an infection has passed. Consequently, IgG antibodies indicate that the subject has had a viral infection in the recent past and has developed antibodies that may protect the subject from future infection.

In order to perform a clinical staging of the subject, i.e. classify the subject by the infection stage, the presence of IgG, the presence of IgM and the presence of pathogens, such as bacteria and/or virus may be used. The clinical staging may result in six different scenarios as shown in the table below.

**Table 1. Patient triage.**

| | Presence of IgM? | Presence of IgG? | Presence of pathogens? | Clinical symptoms? | Staging |
|---|---|---|---|---|---|
| 1 | No | No | yes | | Infection |
| 2 | yes | No | yes | | Early infection |
| 3 | yes | yes | yes | | Later infection |
| 4 | No | yes | No | Yes | Immune system not coping |
| 5 | No | yes | No | No | Coming to end of infection |
| 6 | No | No | No | No | Immune system may have had the virus |

A test strip that is capable of measuring all three parameters, according to table 1, will aid in the triage and allows for a rapid decision of a need for medical intervention. Clinical symptoms of an infection, such as COVID-19, may be taken into account during triage. For example, if a measurement by the lateral flow test strip reveals that the subject has developed IgG antibodies but no IgM or pathogens are detected, the clinical symptoms may be used to classify whether the subject is coming to an end of the infection, or whether the immune system is not capable of eliminating the pathogen, and thereby requires urgent medical intervention.

In yet another embodiment of the present disclosure each test line comprises a different immobilized capturing agent, such as a first capturing agent, a second capturing agent, a third capturing agent and/or a fourth capturing agent. In a specific embodiment of the present disclosure, the lateral flow device has a test line comprising a first capturing agent, another test line comprising a second capturing agent and yet another test line comprising a third capturing agent. In specific examples, the lateral flow device may further comprise even yet a further test line comprising a fourth capturing agent. Typically, the first capturing agent may be selected to capture IgM antibodies, the second capturing agent may be configured for capturing IgG antibodies, the third capturing agent may be configured for capturing pathogen particles, complexes thereof or fragments thereof. The fourth line may be a control line configured to provide a response, typically a visual response, of an accurate measurement.

In a further embodiment of the present disclosure each test line is configured such that it can detect the presence of a different target compound.

In one embodiment of the present disclosure, the lateral flow device is configured for operation as a sandwich assay. A person skilled in the art will appreciate that sandwich assays generally are used for larger compounds since they tend to have multiple binding sites. In a sandwich assay, the sample will come in contact with the conjugate and migrate together towards the test line of the lateral flow device, where a visually present test line indicates the presence of the target compound.

The sandwich assay format is typically used for detecting larger analytes that have at least two binding sites, or epitopes. Usually, an antibody to one binding site is conjugated to the nanoparticle, and an antibody to another binding site is used for the assay's test line. If there is analyte present in the sample, the analyte will bind to both the antibody-nanoparticle conjugate and to the antibody on the test line, yielding a positive signal. The sandwich format results in a signal intensity at the test line that is directly proportional to the amount of analyte present in the sample. Regardless of the quantity of analyte in the sample, an anti-species antibody at the control line will bind the nanoparticle, yielding a strong control line signal that demonstrates that the assay is functioning correctly.

The competitive format is typically used for detecting analytes when antibody pairs are unavailable or if the analyte is too small for multiple antibody binding events, such as steroids and drugs. In this format, the test line typically contains the analyte molecule, usually a protein-analyte complex, and the conjugate pad contains the detection antibody-nanoparticle conjugate. If the target analyte is present, the analyte will bind to the conjugate and prevent it from binding to the analyte at the test line. If the analyte is not present, the conjugates will bind to the analyte at the test line, yielding a signal. In the competitive format, the signal intensity is inversely proportional to the amount of analyte present in the sample. As in the sandwich format, the control line will bind the nanoparticle conjugate with or without the analyte providing confidence that the assay is working correctly. Typically, the conjugate can be said to be a detection particle, as it may, in certain embodiments of the present disclosure, provide an observable signal at a test line regarding the presence of a specific particle, such as a pathogen, in the sample.

In another embodiment of the present disclosure, the lateral flow device is configured for operation as a competitive assay. A person skilled in the art will appreciate that competitive assays generally are used for smaller compounds, but is also preferable for compounds with multiple epitopes.

In another embodiment of the present disclosure, the lateral flow device simultaneously functions as both a sandwich assay and a competitive assay.

In another embodiment of the present disclosure, the membrane layer of said test directly contacts the test line. The immobilized binding agent may thereby be immobilized to a part of the membrane layer.

In another embodiment of the present disclosure, the lateral flow device further comprises an absorbent pad, located at a distal end of the lateral flow device. The absorbent pad may for example be provided as part of the reagent zone. An absorbent pad allows for controlled release of sample and conjugate onto the lateral flow device and to filter undesired components. The absorbent pad may be in any type of material that absorbs water, such as cellulose fibre or woven meshes. Preferably the application pad comprises the conjugate zone and/or the sample loading zone, such as a conjugate pad and/or a sample pad. In a particular embodiment of the present disclosure the membrane layer comprises the reagent zone.

The absorbent pad may be configured to increase the total volume of sample that can enter the test strip. The bed volume of any membrane is finite, and having an absorbent pad at the distal end of the test strip can increase the volume of sample that can be run across the membrane as it acts as a sponge for the additional volume. As such, the presence of an absorbent pad can contribute to the reduction of non-specific binding and sensitivity. This may be accomplished due to the additional volume that can run across the test line washing non-specifically bound material off the test line, and allowing for an increase in totally analyte concentration to reach the test line.

In yet another embodiment of the present disclosure, the lateral flow device further comprises an application pad, and wherein the application pad comprises the conjugate zone and/or the sample loading zone.

In one embodiment of the present disclosure, the lateral flow device comprises a backing layer in an inert material. Inert materials are materials which have minimal or almost no chemical or biological activity when present in the environment.

It is a strong preference that the membrane layer comprises or consists of a porous layer for wicking of a liquid sample. In an embodiment of the present disclosure, the membrane layer of said test comprises or consists of nitrocellulose, cellulose acetate or paper.

In a further embodiment of the present disclosure, the lateral flow device comprises a cassette, and/or wherein the lateral flow device is configured such that it can be operated as a dipstick.

In one embodiment of the present disclosure, the capturing agent, such as any of a first, second, third or fourth capturing agent, of said lateral flow device comprises a capture protein binder. In another embodiment of the present disclosure, said capture protein binder is selected from the list including an antibody, an aptamer, an affimer, an RNA molecule, a DNA molecule, an organic polymer, the target compound, a derivative of the target compound, or a fragment thereof. The capture protein binder may for example comprise a virus particle, or a fragment thereof, for example attached to a colloidal particle at one or more of the test lines. In yet another embodiment of the present disclosure, said organic polymer is a molecularly imprinted polymer.

As disclosed elsewhere herein, the lateral flow test typically comprises multiple test lines, where each test line is configured to capture and/or detect a different compound. In cases wherein the lateral flow device comprises multiple test lines, it is a preference that the device comprises one test line configured for capturing IgM, one test line configured for capturing IgG, one test line configured for capturing a pathogen particle, such as a virus particle. Further, the lateral flow device typically also comprises a test line for providing an indication of an accurate measurement.

In an embodiment of the present disclosure, any of the capturing agents, such as any of a first, second, third or fourth capturing agent, comprises a capture protein binder, typically wherein each capturing agent has a unique capture protein binder. The capture protein binder may be selected from the list including an antibody, an aptamer, an affimer, an RNA molecule, a DNA molecule, an organic polymer, the target compound, a derivative of the target compound or a fragment thereof. The capture protein binder may for example comprise a virus particle, or a fragment thereof, for example attached to a colloidal particle at one or more of the test lines. In yet another embodiment of the present disclosure, said organic polymer is a molecularly imprinted polymer.

In one embodiment of the present disclosure, the capturing agent and/or conjugate of the lateral flow device disclosed herein, is specific for an epitope of the target compound. In another embodiment of the present disclosure, said target compound comprises multiple copies of the epitope.

In one embodiment of the present disclosure, the membrane layer of the lateral flow device disclosed herein, comprises a control line. In another embodiment of the present disclosure, said control line comprises an immobilized capturing agent specific for binding to the conjugate. In yet another embodiment of the present disclosure, said control line comprises the target compound, or a fragment thereof.

In a preferred embodiment of the present disclosure, the lateral flow device comprising a reagent zone (2) and a membrane layer (3). The test lines are here shown as visually observable and comprises a first test line (11), a second test line (12), and a third test line (13). The device furthermore comprises a control line (14). In a specific embodiment of the present disclosure the lateral flow device is targeting triaging of viral infections. For the three test line the first test line is configured to detect the presence of virus in the sample, thereby comprising a capturing agent specific for an epitope of the surface of the virus particle (19), the second test line is configured to detect the presence of IgM, the capturing agent may consequently be an anti-human IgM antibody (20), the third test line is configured to detect the presence of IgG, the capturing agent may consequently be anti-human IgG antibody (21). The control line is configured to target a conjugate, such as a non related control line conjugate, and may consequently be an antibody targeting this conjugate (22).

### Kit

In another aspect, the invention relates to a kit for detecting the presence of a target compound in a sample. The kit may comprise a lateral flow device and reagents. For example the kit may comprise a buffer solution suitable for diluting the sample and obtaining a sample solution. Furthermore, the kit may comprise a lateral flow device comprising a membrane layer. The membrane layer may comprise at least one test line comprising an immobilized capturing agent. Furthermore, the lateral flow device may comprise a reagent zone, such as for receiving sample, conjugates, and/or buffer solution. The reagent zone may comprise a conjugate zone for receiving the running buffer, and/or a sample loading zone for receiving the sample. It is a preference that the sample loading zone is located, along the axial length of the lateral flow device, between the conjugate zone and the at least one test line. Typically the lateral flow device comprises an absorption pad at a distal end of the lateral flow device. Near the opposite end, the proximate end, of the strip, it may be advantageous to have the conjugate zone located. Thereby, a running buffer, if at all used, may be applied to the conjugate zone by dipping the proximate end of the lateral flow device in the buffer solution. It is a preference that the conjugate zone is nearer the proximate end than the sample loading zone.

In one embodiment of the present disclosure, the kit comprises a buffer solution, the buffer solution comprising sodium chloride, bovine serum albumin, and a borate buffer. In one embodiment of the present disclosure, the kit comprises a buffer solution, the buffer solution consisting of sodium chloride, bovine serum albumin, and a borate buffer.

In one embodiment of the present disclosure, the kit comprises a buffer solution, the buffer solution comprising 1 M sodium chloride, 1% w/v bovine serum albumin, and 0.1M borate buffer.

In one embodiment of the present disclosure, the kit comprises a buffer solution, the buffer solution consisting of 1 M sodium chloride, 1% w/v bovine serum albumin, and 0.1M borate buffer.

In one embodiment of the present disclosure, the kit comprises a lateral flow device configured as disclosed elsewhere herein.

In one embodiment of the present disclosure, the conjugate zone of said kit comprises, in the dry unused state, the conjugates. In another embodiment of the present disclosure, said conjugates are provided in a dry state, and upon application of a mobile phase, said conjugates will flow substantially with the mobile phase. Thus, no running buffer may be needed.

In yet another embodiment of the present disclosure, said kit comprises a receptacle comprising the conjugates, as part of a conjugate solution. The lateral flow device is lowered into the conjugate solution in a conjugation well without lowering the sample loading zone in the buffer. The conjugates in the conjugate solution will flow into the membrane and continue along the lateral flow device.

In yet another embodiment of the present disclosure, the kit further comprises a running buffer.

In yet another embodiment of the present disclosure, the conjugates are configured such that upon application of the running buffer, said conjugates will flow substantially with the running buffer, such as from a proximate end of the lateral flow device to a distal end of the lateral flow device.

In yet another embodiment of the present disclosure, the conjugates are provided as part of the running buffer, or such that the conjugates can be mixed with the running buffer before application to the lateral flow device.

In yet another embodiment of the present disclosure, the kit further comprises a receptacle for retaining the running buffer.

In an embodiment of the present disclosure, the kit is configured such that the running buffer may be applied to an end of the lateral flow device, such as the proximate end, by pipetting, and/or such that the running buffer may be applied to the lateral flow device by dipping an end of said lateral flow device, such as the proximate end, in the receptacle.

### Method

In one aspect, the invention relates to a method for detecting the presence of at least one target compound in a sample. The method may comprise the provision of a kit as disclosed herein. The method may comprise the provision of a lateral flow device and reagents. For example the kit may comprise a buffer solution. Furthermore, the method may comprise the provision of a lateral flow device comprising a membrane layer. The membrane layer may comprise at least one test line comprising an immobilized capturing agent. Furthermore, the lateral flow device may comprise a reagent zone, such as for receiving sample, conjugates, and/or buffer solution. The reagent zone may comprise a conjugate zone for receiving the running buffer, although use of a running buffer is not necessary for the method disclosed herein, and/or a sample loading zone for receiving the sample. It is a preference that the sample loading zone is located, along the axial length of the lateral flow device, between the conjugate zone and the at least one test line. Typically the lateral flow device comprises an absorption pad at a distal end of the lateral flow device. Near the opposite end, the proximate end, of the strip, it may be advantageous to have the conjugate zone located. Thereby, the running buffer, if used, may be applied to the conjugate zone by dipping the proximate end of the lateral flow device in the buffer solution. It is a preference that the conjugate zone is nearer the proximate end than the sample loading zone. The method may further comprise the step of applying the sample to the sample loading zone. Preferably the lateral flow device, and the sample volume, is configured and/or selected such that the majority of the sample progress towards the absorption pad, and does not interact with any present conjugates. The method may also comprise the step of mixing the sample with the buffer solution, wherein the volumetric ratio between the buffer solution and the sample is between 5 parts buffer solution and 1 part sample and 15 part buffer solution and 1 part sample, thereby obtaining a sample solution. This step may be important for reducing the viscosity of the sample and allowing a sensitive and specific detection of the at least one target compound. Further, the method may comprise the step of applying the sample solution to the sample loading zone. Further the method may comprise the step of waiting a sufficient amount of time for the sample and the conjugates to flow over the test lines. The sufficient time may for example be until the control line is visually observable. Furthermore, the method may comprise the step of measuring, such as detecting, for each test line, the presence of at least one indicator. The method may thereby be used for detecting the presence of at least one target compound in the sample.

Furthermore, the method may further comprise the step of applying the running buffer to the conjugate zone. Thereby, the running buffer may reconstitute the conjugates, if provided preloaded on the lateral flow device.

In a specific embodiment of the present disclosure, the kit is configured as disclosed elsewhere herein, and/or wherein the lateral flow device is configured as disclosed elsewhere herein.

In a preferred embodiment of the present disclosure, the application of the sample and/or the application of the running buffer is configured such that a substantial fraction of the sample is allowed to flow over the test line before the conjugates.

In one embodiment of the present disclosure, the sample is first allowed to flow over the test lines before loading the conjugate. A person skilled in the art will appreciate that this prevents the conjugate and sample from mixing prior to reaching the test line, thereby avoiding aggregation on the test lines.

### Method 1 - conjugates in solution

In one embodiment of the present disclosure, the lateral flow device of said method is provided without the conjugates present at the conjugate zone.

In another embodiment of the present disclosure, the running buffer of said method comprises conjugates.

### Method 2 - conjugates on lateral flow device

In one embodiment of the present disclosure, the lateral flow device of said method is provided with the conjugates present at the conjugate zone, such as in the dry unused state.

In another embodiment of the present disclosure, the running buffer of said method does not comprise conjugates.

In another embodiment of the present disclosure, said method does not need the use of a running buffer. The presently disclosed method is capable of achieving highly sensitive and specific measurement without the need for a running buffer. Preferably the buffer solution is an aqueous solution and/or wherein the sample is mixed with the buffer solution at a volumetric ratio of 1 part sample to between 2 and 15 parts buffer solution, more preferably between 5 and 10 parts buffer solution, yet more preferably between 9 and 10 parts buffer solution. The buffer solution is advantageously mixed with the sample to obtain a sample solution, and the buffer solution present in the sample solution is sufficient to mobilize the analytes of the sample and/or the conjugates present at the conjugate zone, such as in the dry unused state.

### Sample/conjugate addition and readout

In one embodiment of the present disclosure, the running buffer of said method is added to the loading zone, such as the conjugate zone, and/or a proximate end of the lateral flow device, at or near the conjugate zone.

In an embodiment of the present disclosure, the running buffer is first applied to an area between the sample loading zone and the conjugate zone, followed by a second application of running buffer to the conjugate zone and/or the proximate end of the lateral flow device, wherein the first application is carried out for a predetermined time and/or consists of application of a predetermined volume.

In another embodiment of the present disclosure, said running buffer is applied to the loading zone by dipping an end of the lateral flow device in the running buffer.

In yet another embodiment of the present disclosure, the sufficient time is the time lapsed until the control line is expected to be detectable, such as visually detectable.

In one embodiment of the present disclosure, the indicators of the method disclosed herein, are a number of immobilized conjugates comprising the label, such as forming a visually detectable line.

In another embodiment of the present disclosure, a major fraction of the sample is allowed to migrate from the sample loading zone to the test lines and contact the capturing agents without the presence of conjugates, such as before the conjugates.

In yet another embodiment of the present disclosure, said conjugates are allowed to migrate from the loading zone to the test lines and, if the target compound is bound to the capturing agent and the test line, are present, said conjugates bind to their respective test lines, thereby forming a detectable signal. In one embodiment of the present disclosure, said detectable signal indicates the presence of the target compound in the test sample.

### Infectious disease

In one embodiment of the present disclosure, the target compound of the method disclosed herein, is a biomarker for an infectious disease. In another embodiment of the present disclosure, said infectious disease is a viral disease. In yet another embodiment of the present disclosure, said viral disease is a coronavirus infection. The coronavirus infection may be of the type SARS-CoV-2, also referred to as COVID-19.

In one embodiment of the present disclosure, the membrane layer of the method disclosed herein, comprises a test line that comprises or consists of an immobilized capturing agent specific for IgM antibodies, such as human IgM antibodies, such as a first test line. In another embodiment of the present disclosure, said membrane layer comprises a test line that comprises or consists of an immobilized capturing agent specific for IgG antibodies, such as human IgG antibodies, such as a second test line. In yet another embodiment of the present disclosure, said membrane layer comprises a test line that comprises or consists of an immobilized capturing agent specific for a biomarker of a disease, such as disease-specific antibodies, such as a third test line.

IgG antibodies is the most common type of antibody found in the blood circulation. A person skilled in the art will know that IgM antibodies are the first antibodies to be produced in the response to an initial exposure to an antigen. Therefore, IgM levels in the blood are indicative of an active infection. IgG antibodies, on the other hand, are generated following class switching and maturation of the antibody response, thus they participate predominantly in the secondary immune response, thereby indicating a long-term response to an infection.

### Sample

In one embodiment of the present disclosure, the sample comprises or consists of whole blood, serum, plasma, nasal secretions, sputum, urine, saliva and/or stool.

In one embodiment of the present disclosure, the sample comprises or consists of saliva.

In another embodiment of the present disclosure, the sample has been pre-treated, such as by mixing with a buffered solution, that may comprise anticoagulants. Anticoagulants are additives that inhibit for example blood and/or plasma from clotting, ensuring that the constituent that is to be measured, is non-significantly changed prior to the analytical process.

In another embodiment of the present disclosure, the sample has been pre-treated, such as by mixing with a buffered solution, that comprises sodium chloride, bovine serum albumin, and a borate buffer.

In another embodiment of the present disclosure, the sample has been pre-treated, such as by mixing with a buffered solution, that consists of sodium chloride, bovine serum albumin, and a borate buffer.

In another embodiment of the present disclosure, the sample has been pre-treated, such as by mixing with a buffered solution, that comprises 1 M sodium chloride, 1% w/v bovine serum albumin, and 0.1M borate buffer. It has been found that a buffer comprising at least one, preferably all, these components effectively reduces viscosity of the sample, in particular wherein the sample is saliva, and thereby helps in preventing formation of aggregates within the sample, such as within the sample solution.

In another embodiment of the present disclosure, the sample has been pre-treated, such as by mixing with a buffered solution, that consists of 1 M sodium chloride, 1% w/v bovine serum albumin, and 0.1M borate buffer.

In one embodiment of the present disclosure, the sample is diluted in the buffer solution by mixing between 5 parts buffer solution and 1 part sample and 15 part buffer solution and 1 part sample, thereby obtaining a sample solution.

In one embodiment of the present disclosure, the sample is diluted in the buffer solution by mixing 1 part sample and at least 5 parts buffer solution, more preferably at least 6 parts buffer solution, more preferably at least 7 parts buffer solution, more preferably at least 8 parts buffer solution, even more preferably at least 9 parts buffer solution, most preferably at least 10 parts buffer solution, such as 11 parts buffer solution, or 12 parts buffer solution, or even 13 parts buffer solution, or 14 parts buffer solution, such as 15 parts buffer solution. Preferably, the sample is diluted in the buffer solution by mixing 1 part sample and 10 parts buffer solution.

In one embodiment of the present disclosure, the sample is saliva, and it is diluted in the buffer solution by mixing between 5 parts buffer solution and 1 part sample and 15 part buffer solution and 1 part sample, preferably about 10 parts buffer solution and 1 part sample, thereby obtaining a sample solution.

In one embodiment of the present disclosure, diluting the sample in buffer solution as described herein is important for reducing the viscosity of the sample, for example wherein the sample is saliva, and thus preventing formation of aggregates within the sample, such as within the sample solution.

An important advantage of the method disclosed herein, wherein formation of aggregates within the sample is minimized or avoided, is that there is no or very low non-specific binding to the immobilized capturing agent in the membrane layer. Thus, the detection has high sensitivity and specificity.

In one embodiment of the present disclosure, the membrane layer has not been provided with a blocking agent, such as after plotting the test and/or control line. This helps to minimize the time required for measurement, such to minimize and/or reduce the time sufficient for the sample solution to arrive at the test lines, and for the control line to be detectable, such as visually detectable. A common blocking agent is a solution comprising bovine serum albumin (BSA), which is typically applied to the membrane layer and allowed to dry. Other types of blocking agent are known to the person skilled in the art.

In one embodiment of the present disclosure, the capturing agents are configured for capturing a coronavirus, such as a human coronavirus, or fragments thereof.

In one embodiment of the present disclosure, the capturing agents are configured for binding to a spike protein, such as SARS-CoV S protein and/or SARS-CoV-2 S protein.

Thus, in one embodiment of the present disclosure, the method and/or the kits disclosed herein are suitable for diagnosis of an infectious disease, such as for diagnosis of COVID-19.

### Detailed description of drawings

The invention will in the following be described in greater detail with reference to the accompanying drawings. The drawings are exemplary and are intended to illustrate some of the features of the presently disclosed lateral flow devices, kits, and methods for detecting the presence of at least one target compound in a sample, and are not to be construed as limiting to the presently disclosed invention.

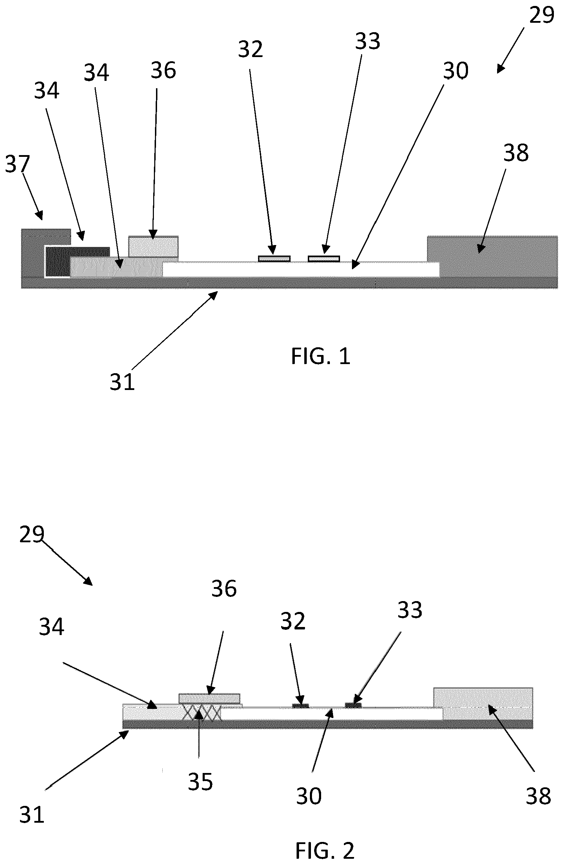

Figure 1 illustrates a lateral flow test device (29) comprised of a nitrocellulose membrane (30) provided on an inert backing card (31). The membrane comprises a test line (32) and may advantageously further comprise a control line (33). The loading zone comprises two sample/conjugate pads (34), wherein at least one comprises conjugates, such as gold conjugates, for enabling a visual line at the test line or control line. Although not essential, the exemplified device comprises a Saliva filter pad (36) for filtering of the saliva sample and/or a sample solution comprising a saliva sample. Typically the saliva filter is a porous structure with pores in the size range between 0.1 and 1 µm. The device further comprises an end pad (37) for preventing leakage. The end pad may be provided, at the proximal end of the device, in a hydrophobic material. At the distal end of the device, across the test lines, is provided an absorbent pad (38).

Figure 2 illustrates a lateral flow test device (29) comprising a nitrocellulose membrane (30), on an inert backing card (31). The membrane comprises a test line (32) and a control line (33). The device has a sample/conjugate pad (34) comprising gold conjugates (35), an (optional) Saliva filter pad (36), and a sink pad/absorbent pad (38).

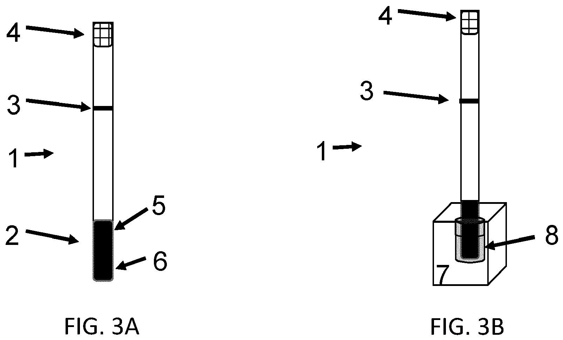

Figure 3 shows schematic representations of a configuration of a lateral flow device according to a specific embodiment of the present disclosure. Fig. 3A shows a schematic representation of a lateral flow device (1) of the present disclosure. The device comprises a reagent zone (2), a membrane layer with a test line (3) and an absorption pad (4). The reagent zone comprising a sample loading zone (6) and a conjugate zone (5). While a sample solution may be applied to the reagent zone by pouring, pipetting or directly from the patient (e.g. spitting), Fig. 3B shows a further example wherein the lateral flow device is dipped into a receptacle (7) comprising a sample solution (8). While the conjugates may be provided as part of the buffer solution, and thereby present in the sample solution before application to the lateral flow device, the conjugates may further be provided together with the device, in the dry unused state, such that the sample solution will, upon application of said solution to the lateral flow device, interact with the conjugates. Once the sample solution is applied to the device, the sample solution will flow from the proximate end of the lateral flow device, to the distal end of the device, i.e. from the reagent zone to the test lines.

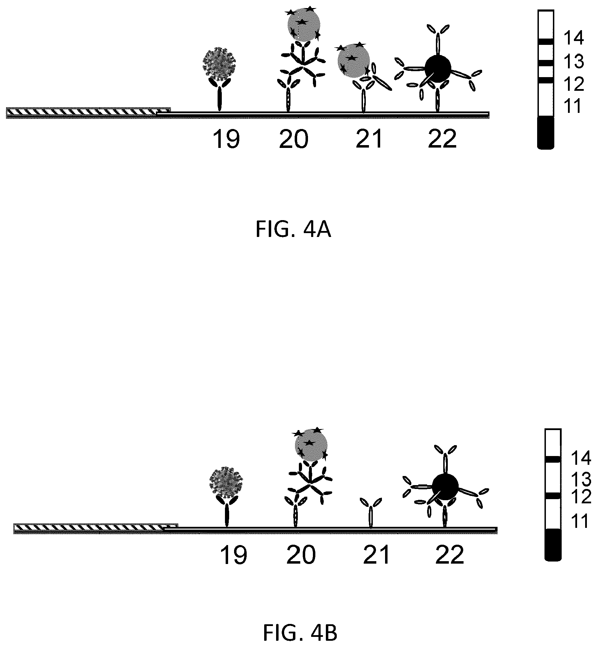

Figure 4 shows various outcomes of a viral infection comprising multiple test lines. In preferred embodiments of the present disclosure, the device comprises at least one test line configured to capture pathogens or fragments thereof, such as a virus particle. The shown device comprises a first test line (11) comprising detection agents in the form of anti-virus antibodies (19), such as anti COVID-19 antibodies, a second test line (12) comprising anti-human IgM antibodies (20), a third test line (13) comprising anti-human IgM antibodies (21), and a control line (14) comprising antibodies directed at capturing non-related control line conjugates (22). Following application of a sample solution and the reconstitution of the conjugates, if provided with the lateral flow device in the dry unused state, the analytes and/or analyte-conjugate complexes are allowed to interact with the test line and/or control line. A visually present control line indicates that the test has operated correctly, the presence of a visually present test line may either indicate the presence of the target compound (analyte) or the absence of the target compound in the sample, depending on the configuration of the specific test line. Either of the one or more test lines may be provided in a competitive or sandwich format. For example, for the first test line as shown in Fig. 4, binding of a virus particle, makes conjugates comprising synthetic fragments of virus as the conjugate protein binder not able to bind to the first test line, thereby producing an absence of a visual line, similar to a competitive assay format. For the second and third test line, the conjugates may comprise conjugate protein binders directed at the respective target compounds, thereby producing visual lines if the target compound is present. While the test lines for human IgG and IgM antibodies function as a sandwich assay, where a visible line indicates the presence of these antibodies in the blood, the test line for the target compound, such as COVID-19, functions as a competitive assay, where a visible line indicates the absence of antibodies against the virus. In Fig. 4A the test line for the virus (11) is not visible, indicating the virus is present. Furthermore, the control line (14) is also visible, as well as the lines for IgM (12) and IgG (13) antibodies. In Fig. 4B, the control line and test line for human IgM antibodies are visible, suggesting an active infection, and that the test subject is infectious.

### Examples

### Example 1 Fabrication of a lateral flow device

### Material and methods:

A lateral flow test device was fabricated by assembling components according to Fig. 1. As shown, the lateral flow test device (29) comprised a nitrocellulose membrane (30), on an inert backing card (31). The membrane comprised a test line (32) and a control line (33). The loading zone comprised two sample/conjugate pads (34) comprising gold conjugates according to the protocol below and an end pad for receiving conjugate buffer (37). At the other end of the device, across the test lines, was an absorbent pad (38). The devices had an optional saliva filter pad (36).

The sample/conjugate pads were pre-treated with a Tris buffered saline solution comprising BSA, PVP and Tween 20. The saliva filter pad was pre-treated with a PBS solution comprising BSA, NaCI and Tween 20. The nitrocellulose membrane was pre-treated with a Tris buffered saline solution comprising BSA, NaCI, BSA, sucrose and PVP.

Gold conjugates were fabricated by the following protocol:
- Obtain gold nanoparticles particles in Di water
- Dissolve anti-2019-nCoV-2 antibodies in the solution
- After all antibody has been added stir for 90 minutes
- Add 2% BSA in Borate buffer pH 7.5
- Stirred 60 minutes
- Centrifuge 6000 rpm for 5 minutes
- Dispose of supernatant and resuspend in relevant OD in 1% BSA and 0.5% Tween

Plotting and blocking of the nitrocellulose membrane was performed by a standard plotter by plotting a solution of anti-SARS-CoV2 nucleocapsid protein mAb mixed with anti-2019-nCoV-2 antibody to give a total antibody concentration of 0.5 mg/ml. The nitrocellulose membrane was blocked by a TBS solution with NaCl, BSA, Sucrose and PVP.

### Conclusion

A lateral flow device for diagnosing COVID-19 by detection of protein antigen from SARS-CoV-2 in saliva samples was fabricated.

### Example 2 Detection of protein antigen from SARS-CoV-2 in saliva samples from individuals suspected of COVID-19

The lateral flow test used was an immuno-chromatographic membrane assay that uses highly sensitive antibodies to detect SARS-CoV-2 Spike protein from saliva specimens. A SARS-CoV-2 specific antibody and a control antibody are immobilized onto a membrane support as two distinct lines and combined with other reagents/pads to construct a test strip, as shown in Fig. 2. The figure shows a lateral flow test device (29) comprising a nitrocellulose membrane (30), on an inert backing card (31). The membrane comprises a test line (32) and a control line (33). The device has a sample/conjugate pad (34) comprising gold conjugates (35), a Saliva filter pad (36), and a sink pad/absorbent pad (38).

The lateral flow test strip consists of:
1. A backing card structure to support the different layers
2. A sample/conjugate pad, where the mobile phase of the assay is sprayed.
3. A nitrocellulose membrane comprising the solid phase of the assay, which comprises two distinct areas, one where the test line is plotted and one where the control line is plotted.
4. A sink pad

Both the sample/conjugate and saliva filter pads are treated in order to compensate the pH effect form the saliva sample and remove or eliminate any non-specific interactions.
The mobile phase consisted of two parts:
1. An anti-2019 nCoV (S1) 40nm gold conjugate and
2. A goat anti-mouse IgG conjugate

Both conjugates are diluted in a gold spraying buffer, containing high amounts of sucrose and protein, in order to enrich the particles' surface and improve the stability of the conjugates. Both gold conjugates are sprayed at a concentration of 10 OD each.

The nitrocellulose (NC) is plotted with anti-2019 nCoV (S1) to form the test line and rabbit anti mouse IgG to form the control line. After the plotting the NC is being blocked in order to remove any non-specific binding and aggregation of the gold conjugate.

A saliva sample is added to the lateral flow device and the test line is visually inspected after 30 minutes.

Subjects that are both positive and negative for COVID-19 infection, as measured with a gold standard method, are tested using the lateral flow test strip. For the subjects that are determined to have an ongoing COVID-19 infection, two visual lines appear shortly after adding the saliva sample to the test line, the first visual line indicating that the virus particles are correctly detected and the second visual line (control line) indicating the validity of the test. For the subjects that are determined to not have an ongoing COVID-19 infection, a single visual line appears shortly after adding the saliva sample to the test line, the single visual line (control line) indicating the validity of the test.

### Conclusion

The lateral flow test is capable of correctly diagnosing COVID-19 by detection of protein antigen from SARS-CoV-2 in saliva samples.

### Example 3 Evaluation of Covid-19 lateral flow test

### LOD and specificity using filtered spiked saliva (using salivate)

### Preparing serial dilutions

The SARS-CoV-2 isolate REMRQ0001/Human/2020/Liverpool is used for the serial dilutions and Vero E6 cells (C1008; African green monkey kidney cells) are used to propagate the virus. Cells are maintained in Dulbecco's minimal essential medium (DMEM) containing 10% foetal bovine serum (FBS) and 0.05 mg/ml gentamicin at 37°C with 5% CO₂ and FBS concentration was reduced to 4% for viral propagation.

Ten-fold serial dilutions of virus stock starting at 10⁶ pfu/ml are made using DMEM media as a diluent. Saliva is collected using salivates. Serial dilutions are made with saliva at a final concentration of 10⁶, 10⁵, 10⁴, 10³ and 10² pfu/ml. Three lots are tested and 10 replicates per lot are used. Saliva of the negative donors is used as negative controls.

### Test evaluation

The measurements are carried out on COVID-19 Saliva lateral flow tests. For the positive samples, a test will be considered a fail if no control line is visible after 30 minutes.

Percentage sensitivity will be calculated by the following formula: Sensitivity = (Observed Positive Results/Actual Positive Results) x100

Intra-lot precision is a measurement of the variation within a single batch of COVID-19 Saliva Tests. It can be calculated using the following formula: Precision Intra-lot=(Observed Results/Expected Results)x100

In this validation protocol the mean results from each lot of assays at each concentration (ran during the validation study for limit of detection and dynamic range) will be compared and percentage standard error calculated for the three lots.

### Evaluation of cross reactivity and interference

The specificity of the COVID-19 saliva test is evaluated by testing potentially cross-reactive microorganisms. The microorganisms are tested in triplicate in the absence (cross-reactivity) or presence of SARS-CoV-2 virus (interference) in saliva samples. The microorganisms include Adenovirus, Human Metapneumovirus (hMPV), Parainfluenza virus 1-4, Influenza A & B, Enterovirus, Respiratory syncytial virus, Rhinovirus, Haemophilus influenzae, Streptococcus pneumoniae, Streptococcus pyogenes, Candida albicans, Pooled human nasal wash, Bordetella pertussis, Mycoplasma pneumoniae, Chlamydia pneumoniae, Legionella pneumophila, Staphylococcus aureus, Staphylococcus epidermidis

### Example 4 Evaluation of Covid-19 virus epitope sensitivity

Lateral flow tests were fabricated comprising a porous phase of nitrocellulose, a sample pad and an absorbent pad. The test line was either sprayed with Anti-2019-n-CoV(S1) targeting the more prominent S protein on the surface of the virus, and Hu mAb to SAR-CoV-2 nucleocapsid protein targeting a less prominent N protein inside the virus.

The test lines were sprayed by a solution comprising either of the two antibodies at various concentrations between 0.1 and 5 mg/ml.

Saliva samples positive for Covid-19 were tested, and it was concluded that devices having a test line directed at the S protein of the virus had better sensitivity than those that were directed at the N protein.

### Example 5 Optimization of Lateral flow tests

Lateral flow tests were fabricated comprising a porous phase of nitrocellulose, a sample pad and an absorbent pad. The test line was sprayed with Anti-2019-n-CoV(S1) targeting the more prominent S protein on the surface of the virus. The control line was Ms mAb to human IgG.

### Optimization of test line and control line concentration

Measurements were performed at multiple concentrations for the test and control line and the most sensitive results were seen at 2.4 mg/ml test line and 2.0 mg/ml control line. These concentrations were acquired by diluting the COVID antibody down to 1.2 mg/ml in PBS 0.1% BSA buffer, then both the test and control line were sprayed down twice. These concentrations showed the best signals and no non-specific binding (NSB) when run with distilled water.

### Optimization of saliva buffer

The saliva mix buffer formula was further optimized by testing buffers with different amounts of protein, salt and detergents. The optimal results were obtained by 0.1M borate buffer with 1M NaCI and 1% BSA. Multiple different formulas were assessed and this one resulting in no measurable non-specific binding. The ratio of the saliva mix buffer was also tested. Despite the high dilution of the saliva, a dissolution ratio between the saliva and buffer of 1:10 worked the best (one part saliva). The results are provided in the table below.

| Saliva-Buffer Ratio | % of strips showing NSB |
|---|---|
| 1:1 Ratio | 100% |
| 1:2 Ratio | 100% |
| 1:3 ratio | 100% |
| 1:4 Ratio | 75% |
| 1:4 Ratio | 70% |
| 1:5 Ratio | 67% |
| 1:6 Ratio | 46% |
| 1:7 Ratio | 22% |
| 1:8 Ratio | 12% |
| 1:9 Ratio | 0% |
| 1:10 Ratio | 0% |

### Optimization of run speed

The time until development of test lines and control lines were measured with lateral flow tests, manufactured according to above, where blocking of the nitrocellulose was performed following plotting of the lines, and were no blocking of the nitrocellulose was performed following plotting of the lines. It was concluded that the lateral flow devices that did not have blocking developed control and test lines significantly faster than the others. At the same time the sensitivity and specificity remained at the same level as with blocking.

### Analysis of lateral flow test sensitivity

Five different saliva samples were spiked with known concentrations of virus and tested in triplicates. All the samples before being diluted were showing non-specific binding. When diluted 1 in 10 in the saliva mix buffer there was no non-specific binding present. The results are provided in the table below.

| Saliva Samples | Virus Concentrations | | | |
|---|---|---|---|---|
| | 10⁶ | 10⁵ | 10⁴ | 0 |
| #1 | Strong positive | Medium positive | Low positive | Negative |
| #2 | Strong positive | Medium positive | Low positive | Negative |
| #3 | Strong positive | Medium positive | Low positive | Negative |
| #4 | Strong positive | Medium positive | Low positive | Negative |
| #5 | Strong positive | Medium positive | Low positive | Negative |

### Items

1. A method for detecting the presence of at least one target compound in a sample, the method comprising:
   - providing a buffer solution;
   - providing a lateral flow device comprising
      i. a membrane layer, comprising at least one test line, the at least one test line including an immobilized capturing agent for capturing the at least one target compound in the sample;
      ii. a sample loading zone for receiving the sample, the sample loading zone in fluidic connection with the test line;
   - mixing the sample with the buffer solution, thereby obtaining a sample solution;
   - applying the sample solution to the sample loading zone;
   - measuring the presence of the at least one target compound in the sample.
2. The method according to item 1, wherein the sample comprises or consists of whole blood, serum, plasma, nasal secretions, sputum, urine, saliva and/or stool, preferably wherein the sample is a saliva sample.
3. The method according to any one of the preceding items, wherein the volumetric ratio between the buffer solution and the sample is between 5 parts buffer solution and 1 part sample and 15 part buffer solution and 1 part sample.
4. The method according to any one of the preceding items, wherein the volumetric ratio between the buffer solution and the sample is 1 part sample and at least 5 parts buffer solution, more preferably at least 6 parts buffer solution, more preferably at least 7 parts buffer solution, more preferably at least 8 parts buffer solution, even more preferably at least 9 parts buffer solution, most preferably at least 10 parts buffer solution, such as 11 parts buffer solution, or 12 parts buffer solution, or even 13 parts buffer solution, or 14 parts buffer solution, such as 15 parts buffer solution.
5. The method according to any one of the preceding items, wherein the buffer solution comprises sodium chloride, bovine serum albumin, and a borate buffer.
6. The method according to item 5, wherein the buffer solution comprises 1 M sodium chloride, 1% w/v bovine serum albumin, and 0.1M borate buffer.
7. The method according to any one of the preceding items, wherein the membrane layer has not been provided with a blocking agent, such as after plotting the test and/or control line.
8. The method according to any one of the preceding items, wherein the capturing agents are configured for capturing a coronavirus, such as a human coronavirus, or fragments thereof.
9. The method according to item 8, wherein the capturing agents are configured for binding to a spike protein, such as SARS-CoV S protein and/or SARS-CoV-2 S protein.
10. The method according to any one of the preceding items, wherein the lateral flow device comprises a conjugate zone, and wherein said conjugate zone comprises in a dry unused state conjugates for binding to the target compound and/or the immobilized capturing agent.
11. The method according to any one of the preceding items, wherein the membrane layer comprises a control line.
12. The method according to any one of the preceding items, wherein the control line comprises a further immobilized capturing agent specific for binding to the conjugate.
13. The method according to any one of the preceding items, further comprising waiting a sufficient amount of time for the sample solution to arrive at the test lines.
14. The method according to any one of the preceding items, wherein the sufficient amount of time is the time lapsed until the control line is expected to be detectable, such as visually detectable.
15. The method according to any one of the preceding items, wherein measuring the presence of the at least one target compound in the sample comprises detecting for each test line, the presence of at least one indicator, such as a visible line.
16. The method according to any one of the preceding items, wherein the indicators are a number of immobilized conjugates comprising the label, such as forming a visually detectable line.
17. The method according to any one of the preceding items, wherein a major fraction of the sample is allowed to migrate from the sample loading zone to the test lines and contact the capturing agents without the presence of conjugates.
18. The method according to any one of the preceding items, wherein the detectable signal indicates the presence of the target compound in the test sample.
19. The method according to any one of the preceding items, wherein the membrane layer comprises a test line that comprises or consists of an immobilized capturing agent specific for IgM antibodies, such as human IgM antibodies, such as a first test line.
20. The method according to any one of the preceding items, wherein the membrane layer comprises a test line that comprises or consists of an immobilized capturing agent specific for IgG antibodies, such as human IgG antibodies, such as a second test line.
21. The method according to any one of the preceding items, wherein the membrane layer comprises a test line that comprises or consists of an immobilized capturing agent specific for a biomarker of a disease, such as disease-specific antibodies, such as a third test line.
22. The method according to any one of the preceding items, wherein the lateral flow device is configured for operation as a sandwich assay and/or as a competitive assay.
23. A kit for detecting the presence of a target compound in a sample, the kit comprising:
   - a buffer solution for diluting the sample, wherein said buffer solution comprises sodium chloride, bovine serum albumin, and a borate buffer; and
   - a lateral flow device comprising:
      i. a membrane layer, comprising at least one test line, the at least one test line comprising an immobilized capturing agent for capturing the at least one target compound in the sample;
      ii. a sample loading zone for receiving the sample, the sample loading zone in fluidic connection with the test line.
24. The kit according to item 22, wherein said kit is suitable for running the method according to any one of items 1 to 21.

## Claims

1. A method for detecting the presence of at least one target compound in a sample, the method comprising:
a. providing a buffer solution;
b. providing a lateral flow device comprising
i. a membrane layer, comprising at least one test line, the at least one test line including an immobilized capturing agent for capturing the at least one target compound in the sample;
ii. a sample loading zone for receiving the sample, the sample loading zone in fluidic connection with the test line;
c. mixing the sample with the buffer solution, wherein the volumetric ratio between the buffer solution and the sample is between 5 parts buffer solution and 1 part sample and 15 part buffer solution and 1 part sample, thereby obtaining a sample solution;
d. applying the sample solution to the sample loading zone;
e. measuring the presence of the at least one target compound in the sample.

2. The method according to claim 1, wherein the sample comprises or consists of sputum and/or saliva.

3. The method according to any one of the preceding claims, wherein the volumetric ratio between the buffer solution and the sample is between 7 parts buffer solution and 1 part sample and 12 part buffer solution and 1 part sample.

4. The method according to any one of the preceding claims, wherein the buffer solution comprises sodium chloride, bovine serum albumin, and a borate buffer.

5. The method according to claim 4, wherein the buffer solution comprises 1 M sodium chloride, 1% w/v bovine serum albumin, and 0.1M borate buffer.

6. The method according to any one of the preceding claims, wherein the membrane layer is not provided with a blocking agent, such as wherein a blocking agent has not been applied to the membrane layer after plotting the test and/or control line.

7. The method according to any one of the preceding claims, wherein the capturing agents are configured for capturing a coronavirus, such as a human coronavirus, or fragments thereof.

8. The method according to claim 7, wherein the capturing agents are configured for binding to a spike protein, such as SARS-CoV S protein and/or SARS-CoV-2 S protein.

9. The method according to any one of the preceding claims, wherein the lateral flow device comprises a conjugate zone, and wherein said conjugate zone comprises, in a dry unused state, conjugates for binding to the target compound and/or the immobilized capturing agent.

10. The lateral flow device according to any one of the preceding claims, wherein the membrane layer comprises a control line, and wherein the control line comprises a further immobilized capturing agent specific for binding to the conjugates.

11. The method according to any one of the preceding claims, wherein the method comprises, before step e), waiting a sufficient amount of time for the sample solution to arrive at the test lines, wherein the sufficient amount of time is the time lapsed until the control line is visible, and/or wherein the sufficient amount of time is the time required for the sample to flow from the sample loading zone to the test line.

12. The method according to any one of the preceding claims, wherein measuring the presence of the at least one target compound in the sample comprises detecting for each test line, the presence of at least one indicator, such as a visible line, and, wherein the indicators are a number of immobilized conjugates comprising the label, such as forming a visually detectable line.

13. The method according to any one of the preceding claims, wherein the lateral flow device comprises a saliva filter covering at least a part of the sample loading zone, wherein the saliva filter is configured to filter out mucus of the sample solution.

14. The method according to any one of the preceding claims, wherein the membrane layer comprises a test line that comprises or consists of an immobilized capturing agent specific for IgM antibodies, such as human IgM antibodies, such as a first test line, and wherein the membrane layer comprises a test line that comprises or consists of an immobilized capturing agent specific for IgG antibodies, such as human IgG antibodies, such as a second test line.

15. The method according to any one of the preceding claims, wherein the membrane layer comprises a test line that comprises or consists of an immobilized capturing agent specific for a biomarker of a disease, such as disease-specific antibodies, such as a third test line.
